# EUROPÄISCHE PATENTSCHRIFT

(11) **EP 1 611 080 B1**
(45) Veröffentlichungstag und Bekanntmachung des Hinweises auf die Patenterteilung: **01.07.2015**
(21) Anmeldenummer: 04722172.6
(22) Anmeldetag: 20.03.2004
(51) Int. Cl.: C07C 57/055, C07C 57/05

(54) **VERFAHREN DER HETEROGEN KATALYSIERTEN PARTIELLEN GASPHASENOXIDATION VON ACROLEIN ZU ACRYLSÄURE**
METHOD FOR THE HETEROGENEOUSLY CATALYZED PARTIAL GAS PHASE OXIDATION OF ACROLEIN INTO ACRYLIC ACID
PROCEDE D'OXYDATION EN PHASE GAZEUSE PARTIELLE, HETEROGENE ET CATALYSEE DE L'ACROLEINE EN ACIDE ACRYLIQUE

(30) Priorität: 25.03.2003 DE 10313211; 05.06.2003 US 475794 P
(43) Veröffentlichungstag der Anmeldung: 04.01.2006
(73) Patentinhaber: BASF SE, 67056 Ludwigshafen (DE)
(72) Erfinder: DIETERLE, Martin, 68167 Mannheim (DE); PETZOLDT, Jochen, 68163 Mannheim (DE); MÜLLER-ENGEL, Klaus, Joachim, 76297 Stutensee (DE); ARNOLD, Heiko, Nanjing 210014 (CN)
(86) Internationale Anmeldenummer: PCT/EP2004/002930
(87) Internationale Veröffentlichungsnummer: WO 2004/085365

(56) Entgegenhaltungen:
- EP-A- 1 164 120
- DE-A- 19 948 523

## Beschreibung

Vorliegende Erfindung betrifft ein Verfahren der heterogen katalysierten partiellen Gasphasenoxidation von Acrolein zu Acrylsäure, bei dem man ein Acrolein, molekularen Sauerstoff und wenigstens ein Inertgas, das zu wenigstens 20% seines Volumens aus molekularem Stickstoff besteht, enthaltendes Reaktionsgasausgangsgemisch, das den molekularen Sauerstoff und das Acrolein in einem molaren Verhältnis O₂:C₃H₄O ≥ 0,5 enthält, in einer Reaktionsstufe so über eine Festbettkatalysatorschüttung, die in zwei räumlich aufeinander folgenden Reaktionszonen A,B angeordnet ist, wobei die Temperatur der Reaktionszone A eine Temperatur im Bereich von 230 bis 320°C und die Temperatur der Reaktionszone B ebenfalls eine Temperatur im Bereich von 230 bis 320°C ist, und deren Aktivmasse wenigstens ein die Elemente Mo und V enthaltendes Multimetalloxid ist, führt, dass sich die Reaktionszone A bis zu einem Umsatz des Acroleins von 45 bis 85 mol-% erstreckt und bei einmaligem Durchgang des Reaktionsgasausgangsgemischs durch die gesamte Festbettkatalysatorschüttung der Acroleinumsatz ≥ 90 mol-% und die Selektivität der Acrylsäurebildung, bezogen auf umgesetztes Acrolein, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht.

Das vorgenannte Verfahren der katalytischen Gasphasenoxidation von Acrolein zu Acrylsäure ist allgemein bekannt (vgl. z.B. DE-A 19910508) und insbesondere als zweite Oxidationsstufe bei der Herstellung von Acrylsäure durch zweistufige katalytische Gasphasenoxidation ausgehend von Propen von Bedeutung. Acrylsäure ist ein bedeutendes Monomeres, das als solches oder in Form eines Alkylesters zur Erzeugung von z.B. als Klebstoffen geeigneten Polymerisaten Verwendung findet.

Neben molekularem Sauerstoff und den Reaktanden enthält das Reaktionsgasausgangsgemisch u.a. deshalb Inertgas, um das Reaktionsgas außerhalb des Explosionsbereiches zu halten.

Eine Zielsetzung einer solchen heterogen katalysierten partiellen Gasphasenoxidation von Acrolein zu Acrylsäure besteht darin, bei einmaligem Durchgang des Reaktionsgasgemisches durch die Reaktionsstufe unter ansonsten vorgegebenen Randbedingungen eine möglichst hohe Ausbeute A^{AA} an Acrylsäure zu erzielen (das ist die Molzahl von zu Acrylsäure umgesetztem Acrolein, bezogen auf die Molzahl an eingesetztem Acrolein).

Eine weitere Zielsetzung einer solchen heterogen katalysierten partiellen Gasphasenoxidation von Acrolein zu Acrylsäure besteht darin, eine möglichst hohe Raum-Zeit-Ausbeute (RZA^{AA}) an Acrylsäure zu erzielen (das ist bei einer kontinuierlichen Verfahrensweise die je Stunde und Volumen der verwendeten Festbettkatalysatorschüttung in Litern erzeugte Gesamtmenge an Acrylsäure).

Bei gleich bleibender gegebener Ausbeute A^{AA} ist die Raum-Zeit-Ausbeute um so größer, je größer die Belastung der Festbettkatalysatorschüttung der Reaktionsstufe mit Acrolein ist (darunter wird die Menge an Acrolein in Normlitern (= NI; das Volumen in Litern, das die entsprechende Acroleinmenge bei Normalbedingungen , d.h. bei 25°C und 1 bar, einnehmen würde) verstanden, die als Bestandteil des Reaktionsgasausgangsgemisches pro Stunde durch einen Liter an Festbettkatalysatorschüttung geführt wird).

Die Lehren der Schriften WO 01/36364, DE-A 19927624, DE-A 19948248, DE-A 19948523, DE-A 19948241 und DE-A 19910506 sind daher darauf gerichtet, bei im wesentlichen gleich bleibender A^{AA} die Acroleinbelastung der Festbettkatalysatorschüttung der Reaktionsstufe mit Acrolein signifikant zu erhöhen. Dies gelingt im wesentlichen dadurch, dass die Festbettkatalysatorschüttung in der Reaktionsstufe in jeweils zwei räumlich aufeinander folgenden Temperaturzonen (Reaktionszonen) angeordnet wird. Die Acroleinbelastung der Festbettkatalysatorschüttung wird dabei zu ≥ 150 NI/I Festbettkatalysatorschüttungh gewählt und die Temperatur der jeweils zweiten (in Strömungsrichtung des Reaktionsgasgemisches) Temperaturzone muss wenigstens 10°C oberhalb der Temperatur der ersten Temperaturzone liegen.

In ähnlicher Weise lehrt auch die EP-A 1106598 ein Verfahren der Hochlastfahrweise für die heterogen katalysierte partielle Gasphasenoxidation von Acrolein zu Acrylsäure, bei dem die Festbettkatalysatorschüttung der Reaktionsstufe in mehreren Temperaturzonen angeordnet ist. Dabei kann der Temperaturunterschied einer in Strömungsrichtung des Reaktionsgasgemisches nachfolgenden Temperaturzone gemäß der Lehre der EP-A 1106598 sowohl mehr als auch weniger als 5°C oberhalb der Temperatur der vorhergehenden Temperaturzone liegen, wobei es die EP-A 1106598 völlig offen lässt, unter welchen Bedingungen ein größerer und unter welchen Bedingungen ein kleinerer Temperaturunterschied angewendet werden sollte.

Auch lässt die EP-A 1106598 völlig offen, was unter der Temperatur einer Reaktions- bzw. Temperaturzone zu verstehen ist.

Im Unterschied dazu wird in den übrigen Schriften des zitierten Standes der Technik unter der Temperatur einer Reaktionszone die Temperatur der in der Reaktionszone befindlichen Festbettkatalysatorschüttung bei Ausübung des Verfahrens in Abwesenheit einer chemischen Reaktion verstanden. Ist diese Temperatur innerhalb der Reaktionszone nicht konstant, so meint der Begriff Temperatur einer Reaktionszone hier den (Zahlen)mittelwert der Temperatur der Festbettkatalysatorschüttung längs der Reaktionszone. Wesentlich ist dabei, dass die Temperierung der einzelnen Reaktionszonen im wesentlichen unabhängig voneinander erfolgt, weshalb einer Reaktionszone stets eine Temperaturzone entspricht. Vorstehende Definition der Temperatur einer Reaktionszone soll auch in dieser Schrift gelten.

Da die heterogen katalysierte partielle Gasphasenoxidation von Acrolein zu Acrylsäure eine ausgeprägt exotherme Reaktion ist, ist die Temperatur des Reaktionsgasgemisches beim reaktiven Durchgang durch die Festbettkatalysatorschüttung in der Regel von der Temperatur einer Reaktionszone verschieden. Sie liegt Normalerweise oberhalb der Temperatur der Reaktionszone und durchläuft innerhalb einer Reaktionszone in der Regel ein Maximum (Heißpunktmaximum) oder fällt von einem Maximalwert ausgehend ab.

Nachteilig an den Lehren des Standes der Technik ist jedoch, dass sie ausschließlich darauf gerichtet sind, eine Mehrzonenanordnung unter hoher Acroleinlast zu betreiben. Dies ist insofern nachteilig, als mit einer solchen Verfahrensweise unabdingbar eine hohe RZA^{AA} einhergeht. Diese setzt aber einen entsprechenden Marktbedarf an Acrylsäure voraus. Ist letzterer nicht gegeben (z.B. temporär), muss die Mehrzonenanordnung in notwendiger Weise bei geringeren Acroleinlasten betrieben werden, wobei dann außerdem eine möglichst hohe Selektivität der Acrylsäurebildung, bezogen auf umgesetztes Acrolein, als anzustrebende Zielgröße im Vordergrund steht (S^{AA}). Das ist die bei einmaligem Durchgang durch die Mehrzonenanordnung gebildete molare Menge an Acrylsäure bezogen auf die Molzahl an dabei umgesetztem Acrolein.

Die Aufgabe der vorliegenden Erfindung bestand daher darin, ein Verfahren der heterogen katalysierten partiellen Gasphasenoxidation von Acrolein zu Acrylsäure in einer Mehrzonenanordnung zur Verfügung zu stellen, bei dem bei Acroleinlasten von < 150 Nl/l·h die Acrylsäurebildung mit möglichst hoher Selektivität erfolgt.

Demgemäß wurde ein Verfahren der heterogen katalysierten partiellen Gasphasenoxidation von Acrolein zu Acrylsäure, bei dem man ein Acrolein, molekularen Sauerstoff und wenigstens ein Inertgas, das zu wenigstens 20% seines Volumens aus molekularem Stickstoff besteht, enthaltendes Reaktionsgasausgangsgemisch, das den molekularen Sauerstoff und das Acrolein in einem molaren Verhältnis O₂:C₃H₄O ≥ 0,5 enthält, in einer Reaktionsstufe so über eine Festbettkatalysatorschüttung, die in zwei räumlich aufeinander folgenden Reaktionszonen A,B angeordnet ist, wobei die Temperatur der Reaktionszone A eine Temperatur im Bereich von 230 bis 320°C und die Temperatur der Reaktionszone B ebenfalls eine Temperatur im Bereich von 230 bis 320°C ist, und deren Aktivmasse wenigstens ein die Elemente Mo und V enthaltendes Multimetalloxid ist, führt, dass sich die Reaktionszone A bis zu einem Umsatz des Acroleins von 45 bis 85 mol-% erstreckt und bei einmaligem Durchgang des Reaktionsgasausgangsgemischs durch die gesamte Festbettkatalysatorschüttung der Acroleinumsatz ≥ 90 mol% und die Selektivität der Acrylsäurebildung, bezogen auf umgesetztes Acrolein, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht, gefunden, das dadurch gekennzeichnet ist, dass
a) die Belastung der Festbettkatalysatorschüttung mit dem im Reaktionsgasausgangsgemisch enthaltenen Acrolein ≤ 125 NI Acrolein/l Festbettkatalysatorschüttung h und ≥ 70 NI Acrolein/l Festbettkatalysatorschüttung h beträgt,
b) die volumenspezifische Aktivität der Festbestkatalysatorschüttung in Strömungsrichtung des Reaktionsgasgemisches über die Festbettkatalysatorschüttung entweder konstant ist, oder wenigstens einmal zunimmt, und
c) die Differenz T^{maxA} - T^{maxB}, gebildet aus der höchsten Temperatur T^{maxA}, die das Reaktionsgasgemisch innerhalb der Reaktionszone A aufweist, und der höchsten Temperatur T^{maxB}, die das Reaktionsgasgemisch innerhalb der Reaktionszone B aufweist, ≥ 0°C beträgt.

Mit Vorteil nimmt die volumenspezifische Aktivität der Festbettkatalysatorschüttung in Strömungsrichtung wenigstens einmal zu.

In der Regel wird beim erfindungsgemäßen Verfahren die Differenz T^{maxA} - T^{maxB} nicht mehr als 75°C betragen. Erfindungsgemäß bevorzugt beträgt T^{maxA} - T^{maxB} ≥ 3°C und ≤ 60°C. Ganz besonders bevorzugt beträgt T^{maxA} - T^{maxB} beim erfindungsgemäßen Verfahren ≥ 5°C und ≤ 40°C.

Das erfindungsgemäße Verfahren erweist sich z.B. als vorteilhaft, wenn die Belastung der Festbettkatalysatorschüttung mit dem im Reaktionsgasausgangsgemisch enthaltenen Acrolein ≥ 80 NI Acrolein/l·h und ≤ 125 NI Acrolein/l·h, oder ≥ 90 NI Acrolein/l·h und ≤ 125 NI Acrolein/l·h, oder ≥ 100 Nl Acrolein/l·h und ≤ 120 Nl Acrolein/l·h, oder ≥ 105 Nl Acrolein/l·h und ≤ 115 Nl Acrolein/l·h beträgt.

Selbstverständlich kann das erfindungsgemäße Verfahren auch dann angewendet werden, wenn die Belastung der Festbettkatalysatorschüttung mit dem im Reaktionsgasgemisch enthaltenen Acrolein < 70 Nl Acrolein/l·h beträgt und/oder die volumenspezifische Aktivität der Festbettkatalysatorschüttung konstant ist. Der Betrieb einer Mehrzonenanordnung bei solchermaßen geringen Reaktandenlasten wäre jedoch kaum noch wirtschaftlich.

Die erfindungsgemäß geforderten Differenzen T^{maxA} - T^{maxB} stellen sich bei der Ausübung des erfindungsgemäßen Verfahrens normalerweise dann ein, wenn einerseits sowohl die Temperatur der Reaktionszone A als auch die Temperatur der Reaktionszone B im Bereich von 230 bis 320°C liegt und andererseits die Differenz zwischen der Temperatur der Reaktionszone B (T_{B}) und der Temperatur der Reaktionszone A (T_{A}), d.h., T_{B}-T_{A}, ≤ 0°C und ≥ -20°C oder ≥ -10°C, bzw. ≤ 0°C und ≥ -5°C, oder häufig ≤ 0°C und ≥ -3°C beträgt.

D.h., im Gegensatz zur Lehre des Standes der Technik für hohe Lasten, wird beim erfindungsgemäßen Verfahren die Temperatur der Nachfolgezone normalerweise geringer sein als die Temperatur der vorausgehenden Reaktionszone.

Die vorgenannte Aussage betreffend die Temperaturdifferenzen T_{B}-T_{A} gilt auch dann, wenn die Temperatur der Reaktionszone A im bevorzugten Bereich von 250 bis 300°C bzw. im besonders bevorzugten Bereich von 260 bis 280°C liegt.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1 bis 3 bar liegen. Normalerweise wird der Reaktionsdruck 100 bar nicht überschreiten.

Erfindungsgemäß bevorzugt erstreckt sich die Reaktionszone A bis zu einem Umsatz des Acroleins von 50 bis 85 mol-% bzw. 60 bis 85 mol-%.

In der Regel kann der auf den einfachen Durchgang bezogene Acroleinumsatz beim erfindungsgemäßen Verfahren ≥ 92 mol-%, oder ≥ 94 mol-%, oder ≥ 96 mol-%, oder ≥ 98 mol-% und häufig sogar ≥ 99 mol-% betragen. Die Selektivität der Acrylsäurebildung wird dabei regelmäßig ≥ 92 mol-%, bzw. ≥ 94 mol-%, häufig ≥ 95 mol-% oder ≥ 96 mol-% bzw. ≥ 97 mol-% betragen.

Das molare Verhältnis von O₂:Acrolein im Reaktionsgasausgangsgemisch muß erfindungsgemäß ≥ 0,5 betragen. Häufig liegt es bei Werten ≥ 1. Üblicherweise wird dieses Verhältnis bei Werten ≤ 3 liegen. Häufig beträgt das molare Verhältnis von O₂:Acrolein im Reaktionsgasausgangsgemisch erfindungsgemäß 1 bis 2 bzw. 1 bis 1,5.

Als Katalysatoren für die Festbettkatalysatorschüttung der erfindungsgemäßen gasphasenkatalytischen Acroleinoxidation kommen alle diejenigen in Betracht, deren Aktivmasse wenigstens ein Mo und V enthaltendes Multimetalloxid ist. Solchermaßen geeignete Mulitmetalloxidkatalysatoren können beispielsweise der US-A 3 775 474, der US-A 3954855, der US-A 3893951 und der US-A 4339355 entnommen werden. Ferner eignen sich in besonderer Weise die Multimetalloxidmassen der EP-A 427508, der DE-A 2909671, der DE-C 3151805, der DE-AS 2626887, der DE-A 4302991, der EP-A 700893, der EP-A 714700 und der DE-A 19736105 sowie der DE-A 10046928.

Geeignet sind in diesem Zusammenhang auch die beispielhaften Ausführungsformen der EP-A 714700 sowie der DE-A 19736105.

Eine Vielzahl der für die Festbettkatalysatorschüttung geeigneten Multimetalloxidaktivmassen, z.B. diejenigen der DE-A 19815281, lässt sich unter der allgemeinen Formel IV

Mo₁₂VₐX¹_{b}X²_{c}X³_{d}X⁴ₑX⁵_{f}X⁶_{g}Oₙ (IV),

in der die Variablen folgende Bedeutung haben:
- X¹: = W, Nb, Ta, Cr und/oder Ce,
- X²: = Cu, Ni, Co, Fe, Mn und/oder Zn,
- X³: = Sb und/oder Bi,
- X⁴: = eines oder mehrere Alkalimetalle,
- X⁵: = eines oder mehrere Erdalkalimetalle,
- X⁶: = Si, Al, Ti und/oder Zr,

- a: = 1 bis 6,
- b: = 0,2 bis 4,
- c: = 0,5 bis 18,
- d: = 0 bis 40,
- e: = 0 bis 2,
- f: = 0 bis 4,
- g: = 0 bis 40 und
- n: = eine Zahl, die durch die Wertigkeit und Häufigkeit der von Sauerstoff verschiedenen Elemente in IV bestimmt wird,
subsummieren.

Erfindungsgemäß bevorzugte Ausführungsformen innerhalb der aktiven Multimetalloxide IV sind jene, die von den nachfolgenden Bedeutungen der Variablen der allgemeinen Formel IV erfasst werden:
- X¹: = W, Nb, und/oder Cr,
- X²: = Cu, Ni, Co, und/oder Fe,
- X³: = Sb,
- X⁴: = Na und/oder K,
- X⁵: = Ca, Sr und/oder Ba,
- X⁶: = Si, Al, und/oder Ti,

- a: = 1,5 bis 5,
- b: = 0,5 bis 2,
- c: = 0,5 bis 3,
- d: = 0 bis 2,
- e: = 0 bis 0,2,
- f: = 0 bis 1 und
- n: = eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V

Mo₁₂Vₐ,Y¹_{b},Y²_{c},Y⁵_{f},Y⁶g,Oₙ, (V)

mit
- Y¹: = W und/oder Nb,
- Y²: = Cu und/oder Ni,
- Y⁵: = Ca und/oder Sr,
- Y⁶: = Si und/oder Al,

- a': = 2 bis 4,
- b': = 1 bis 1,5,
- c': = 1 bis 3,
- f': = 0 bis 0,5
- g': = 0 bis 8 und
- n': = eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in an sich bekannter, z.B. in der DE-A 4335973 oder in der EP-A 714700 offenbarter, Weise erhältlich.

Prinzipiell können für die Katalysatoren der Festbettkatalysatorschüttung geeignete Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel IV, werden in der Regel nicht in Pulverform sondern zu bestimmten Katalysatorgeometrien geformt in der Festbettkatalysatorschüttung eingesetzt, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katälysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zircondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für die Katalysatoren der Festbettkatalysatorschüttung zu verwendende Multimetalloxidaktivmassen sind ferner Massen der allgemeinen Formel VI,

[D]ₚ[E]_{q} (VI),

in der die Variablen folgende Bedeutung haben:
- D: = MO₁₂V_{a"}Z¹_{b"}Z²_{c"}Z³_{d"}Z⁴_{e"}Z⁵_{f"}Z⁶_{g"}O_{x"},
- E: = Z⁷₁₂Cu_{h"}H_{i"}O_{y"},
- Z¹: = W, Nb, Ta, Cr und/oder Ce,
- Z²: = Cu, Ni, Co, Fe, Mn und/oder Zn,
- Z³: = Sb und/oder Bi,
- Z⁴: = Li, Na, K, Rb, Cs und/oder H
- Z⁵: = Mg, Ca, Sr und/oder Ba,
- Z⁶: = Si, Al, Ti und/oder Zr,
- Z⁷: = Mo, W, V, Nb und/oder Ta, vorzugsweise Mo und/oder W

- a": = 1 bis 8,
- b": = 0,2 bis 5,
- c": = 0 bis 23,
- d": = 0 bis 50,
- e": = 0 bis 2,
- f": = 0 bis 5,
- g": = 0 bis 50,
- h": = 4 bis 30,
- i": = 0 bis 20 und
- x",y": = Zahlen, die durch die Wertigkeit und Häufigkeit der von Sauerstoff verschiedenen Element in VI bestimmt werden und
- p,q: = von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h"}H_{i"}Oy" (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo¹²V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen VI-Katalysatoren das bei den Multimetalloxidmassen IV-Katalysatoren Gesagte.

Darüber hinaus eignen sich als für die Katalysatoren der Festbettkatalysatorschüttung geeignete Multimetalloxidmassen jene der DE-A 19815281, insbesondere alle beispielhaften Ausführungsformen aus dieser Schrift. Hinsichtlich der Formgebung gilt das vorstehend Gesagte.

Für die Festbettkatalysatorschüttung des erfindungsgemäßen Verfahrens besonders geeignete Katalysatoren sind die Schalenkatalysatoren S1 (Stöchiometrie: MO₁₂V₃W_{1,2}Cu_{2,4}Oₙ) und S7 (Stöchiometrie: Mo₁₂V₃W_{1,2}Cu_{1,6}Ni_{0,8}Oₙ) aus der DE-A 4442346 mit einem Aktivmassenanteil von 27 Gew.-% und einer Schalendicke von 230 µm, der Schalenkatalysator aus Herstellungsbeispiel 5 der DE-A 10046928 (Stöchiometrie: Mo₁₂V₃W_{1,2}Cu_{2,4}Oₙ) mit einem Aktivmassenanteil von 20 Gew.-%, die Schalenkatalysatoren gemäß den Beispielen 1 bis 5 aus der DE-A 19815281, jedoch ebenso wie die vorstehend genannten Schalenkatalysatoren für die zweite Reaktionsstufe auf Trägerringe der Geometrie 7 mm x 3 mm x 4 mm (Außendurchmesser x Länge x Innendurchmesser) mit einem Aktivmassenanteil von 20 Gew.-% (bezogen auf die Gesamtmasse des Schalenkatalysators) aufgebracht, sowie ein Schalenkatalysator mit zweiphasiger Aktivmasse der Stöchiometrie (Mo_{10,4}V₃W_{1,2}Oₓ) (CuMo_{0,5}W_{0,5}0₄)_{1,6}, und hergestellt gemäß der DE-A 19736105 und einem Aktivmassenanteil von 20 Gew.-% auf den vorgenannten 7 mm x 3 mm x 4 mm Träger aufgebracht.

Die vorstehend für die erfindungsgemäße Reaktionsstufe empfohlenen Katalysatoren sind für die erfindungsgemäße Reaktionsstufe aber auch dann geeignet, wenn man alles beibehält und nur die Trägergeometrie auf 5 mm x 3 mm x 1,5 mm abändert (Auβendurchmesser x Länge x Innendurchmesser). Ferner können die genannten Multi-metalloxide aber auch in Form der entsprechenden Vollkatalysatorringe in der erfindungsgemäßen Reaktionsstufe eingesetzt werden.

Zur Bereitung der Festbettkatalysatorschüttung können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungskörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die Festbettkatalysatorschüttung nicht verändert. D.h., die für einen einzelnen Katalysatorformkörper verwendete Aktivmasse kann zwar ein Gemisch aus verschiedenen die Elemente Mo und V enthaltenden Multimetalloxiden sein, für alle Katalysatorformkörper der Festbettkatalysatorschüttung ist dann jedoch das gleiche Gemisch zu verwenden.

Die volumenspezifische (d.h., die auf die Einheit des Volumens normierte) Aktivität kann in diesem Fall z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkende Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für die Festbettkatalysatorschüttung aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Im Normalfall wird beim erfindungsgemäßen Verfahren innerhalb der Festbettkatalysatorschüttung in Strömungsrichtung des Reaktionsgasgemisches die volumenspezifische Aktivität nicht einmal abnehmen.

Vorab und/oder im Anschluss an die Festbettkatalysatorschüttung können sich ausschließlich aus Inertmaterial (z.B. nur Verdünnungsformkörpern) bestehende Schüttungen befinden (sie werden in dieser Schrift begrifflich nicht der Festbettkatalysatorschüttung zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Erfindungsgemäß bevorzugt ist beim erfindungsgemäßen Verfahren die Festbettkatalysatorschüttung in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone der Festbettkatalysatorschüttung befindet sich dann erfindungsgemäß vorteilhaft bis zum Ende der Länge der Festbettkatalysatorschüttung (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn in der Festbettkatalysatorschüttung als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungskörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende der Festbettkatalysatorschüttung.

In anwendungstechnisch zweckmäßiger Weise erfolgt die Durchführung der Reaktionsstufe des erfindungsgemäßen Verfahrens in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A-2201528, der EP-A 383224 und der DE-A 2903218 offenbarten Zweizonenrohrbündelreaktoren sind für eine Durchführung der Reaktionsstufe des erfindungsgemäßen Verfahrens geeignet.

D.h., in einfachster Weise befindet sich die erfindungsgemäß zu verwendende Festbettkatalysatorschüttung (eventuell mit vor- und/oder nach angeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone.

D.h., in einfacher Weise umströmt z.B. ein Salzbad A diejenigen Abschnitte der Rohre (die Reaktionszone A), in welchem sich die oxidative Umsetzung des Acroleins (beim einfachen Durchgang) bis zum Erreichen eines Umsatzwertes im Bereich von 45 bis 85 mol-% (bevorzugt 50 bis 85 mol-%, besonders bevorzugt 60 bis 85 mol-%) vollzieht und ein Salzbad B umströmt den Abschnitt der Rohre (die Reaktionszone B), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Reaktionszone A, B weitere Reaktionszonen anschließen, die auf individuellen Temperaturen gehalten werden).

Anwendungstechnisch zweckmäßig umfasst die Reaktionsstufe des erfindungsgemäßen Verfahrens keine weiteren Reaktionszonen. D.h., das Salzbad B umströmt zweckmäßig den Abschnitt der Rohre, in welchem sich die oxidative Anschlussumsetzung des Acroleins (beim einfachen Durchgang) bis zu einem Umsatzwert von ≥ 92 mol-%, oder ≥ 94 mol-% oder ≥ 96 mol-% oder ≥ 98 mol-% und häufig sogar ≥ 99 mol-% oder mehr vollzieht.

Üblicherweise liegt der Beginn der Reaktionszone B hinter dem Heißpunktmaximum der Reaktionszone A.

Die beiden Salzbäder A, B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstredend kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohr-bündel ein mäanderförmiger Strömungsverlauf des Wärmeaustauschmittels resultiert.

Üblicherweise sind in den vorgenannten Zweizonen-Rohrbündelreaktoren für die erfindungsgemäße Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Ihre Länge beträgt zweckmäßig 3 bis 4, bevorzugt 3,5 m. In jeder Temperaturzone belegt die Festbettkatalysatorschüttung wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündeireaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die so genannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der erfindungsgemäßen Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Reaktionszone bis zur Austrittsstelle aus der Reaktionszone um 0 bis 15°C ansteigt. D.h., das vorgenannte ΔT kann erfindungsgemäß 1 bis 103°C, oder 2 bis 8°C oder 3 bis 6°C betragen.

Die Eintrittstemperatur des Wärmeaustauschmittels in die Reaktionszone A liegt erfindungsgemäß normalerweise im Bereich von 230 bis 320°C, bevorzugt im Bereich von 250 bis 300°C und besonders bevorzugt im Bereich von 260 bis 280°C. Die Eintrittstemperatur des Wärmeaustauschmittels in die Reaktionszone B liegt erfindungsgemäß normalerweise ebenfalls im Bereich von 230 bis 320°C, bzw. bis 280°C, gleichzeitig aber normalerweise ≥ 0°C bis ≤ 20°C oder bis ≤ 10°C, bzw. ≥ 0°C und ≤ 5°C, oder häufig ≥ 0°C und ≤ 3°C unterhalb der Eintrittstemperatur des in die Reaktionszone A eintretenden Wärmeaustauschmittels.

Es sei an dieser Stelle auch noch einmal darauf hingewiesen, dass für eine Durchführung der erfindungsgemäßen Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszonen B eine Teilmenge an die Reaktionszone A abzuführen, um gegebenenfalls ein Anwärmen eines zu kalten Reaktionsgasausgangsgemisches oder eines kalten Kreisgases zu bewirken. Ferner kann die Rohrbündelcharakteristik innerhalb einer individuellen Reaktionszone wie in der EP-A 382 098 beschrieben gestaltet werden.

Normalerweise wird beim erfindungsgemäßen Verfahren Acrolein eingesetzt, das durch katalytische Gasphasenoxidation von Propen erzeugt wurde. In der Regel werden die Acrolein enthaltenden Reaktionsgase dieser Propenoxidation ohne Zwischenreinigung eingesetzt, weshalb das erfindungsgemäße Reaktionsgasausgangsgemisch auch geringe Mengen von z.B. nicht umgesetztem Propen oder von Nebenprodukten der Propenoxidation enthalten kann. Normalerweise muss dabei dem Produktgasgemisch der Propenoxidation noch der für die Acroleinoxidation erforderliche Sauerstoff zugesetzt werden.

Mit Vorteil wird eine solche dem erfindungsgemäßen Verfahren vorausgehende gasphasenkatalytische Oxidation von Propen zu Acrolein in Analogie zum erfindungsgemäßen Verfahren so durchgeführt, dass man ein Propen, molekularen Sauerstoff und wenigstens ein Inertgas enthaltendes Reaktionsgasausgangsgemisch, das den molekularen Sauerstoff und das Propen in einem molaren Verhältnis O₂ : C₃H₆ ≥ 1 enthält, in einer Reaktionsstufe so über eine Festbettkatalysatorschüttung, die in zwei räumlich aufeinander folgenden Reaktionszonen A', B' angeordnet ist, wobei die Temperatur der Reaktionszone A' eine Temperatur im Bereich von 290 bis 380°C und die Temperatur der Reaktionszone B' ebenfalls eine Temperatur im Bereich von 290 bis 380°C ist, und deren Aktivmasse wenigstens ein die Elemente Mo, Fe und Bi enthaltendes Multimetalloxid ist, führt, dass sich die Reaktionszone A' bis zu einem Umsatz des Propens von 40 bis 80 mol.-% erstreckt und der Propenumsatz bei einmaligem Durchgang durch die Festbettkatalysatorschüttung 1 ≥ 90 mol.-% und die damit einhergehende Selektivität der Acroleinbildung sowie der Acrylsäurenebenproduktbildung zusammengenommen ≥ 90 mol.-% betragen, das dadurch gekennzeichnet ist, dass
a) die Belastung der Festbettkatalysatorschüttung mit dem im Reaktionsgasausgangsgemisch enthaltenen Propen ≤ 160 Nl Propen / l Festbettkatalysatorschüttung 1 · h und ≥ 90 Nl Propen / I Festbettkatalysatorschüttung 1 · h beträgt;
b) die volumenspezifische Aktivität der Festbettkatalysatorschüttung in Strömungsrichtung des Reaktionsgasgemisches über die Festbettkatalysatorschüttung entweder konstant ist oder wenigstens einmal zunimmt, und
c) die Differenz T^{maxA'} - T^{maxB'}, gebildet aus der höchsten Temperatur T^{maxA'}, die das Reaktionsgasgemisch innerhalb der Reaktionszone A' aufweist, und der höchsten Temperatur T^{maxB'}, die das Reaktionsgasgemisch innerhalb der Reaktionszone B aufweist, ≥ 0°C beträgt.

In der Regel wird die Differenz T^{maxA'} - T^{maxB'} nicht mehr als 80°C betragen.
Bevorzugt beträgt dabei T^{maxA'} - T^{maxB'} ≥ 3°C und ≤ 70°C. Ganz besonders bevorzugt beträgt dabei T^{maxA'} - T^{maxB'} ≥ 20°C und ≤ 60°C.

Dabei erweist es sich als vorteilhaft, wenn die Belastung der Festbettkatalysatorschüttung mit dem im Reaktionsgasausgangsgemisch enthaltenen Propen ≥ 90 Nl Propen/l h und ≤ 155 Nl Propen/l · h, oder ≥ 100 Nl Propen/l · h und ≤ 150 Nl Propen/l · h, oder ≥ 110 Nl Propen/l · h und ≤ 145 Nl Propen/ l · h, oder ≥ 120 Nl Propen/l · h und ≤ 140 Nl Propen/ l · h, oder ≥ 125 Nl Propen/l · h und ≤ 135 Nl Propen/l · h beträgt.

Die dabei geforderten Differenzen T^{maxA'} - T^{maxB'} stellen sich bei der Ausübung des Verfahrens normalerweise dann ein, wenn einerseits sowohl die Temperatur der Reaktionszone A' als auch die Temperatur der Reaktionszone B' im Bereich von 290 bis 380°C liegt und andererseits die Differenz zwischen der Temperatur der Reaktionszone B' (T_{B'}) und der Temperatur der Reaktionszone A' (T_{A'}), d.h., T_{B'} - T_{A'}, ≤ 0°C und ≥ - 10°C, bzw. ≤ 0°C und ≥ -5°C, oder häufig ≤ 0°C und ≥ -3°C beträgt.

D.h., zur Durchführung des erfindungsgemäßen Verfahrens können zwei Zweizonenrohrbündelreaktoren hintereinander geschaltet oder zu einem Vierzonenrohrbündelreaktor verschmolzen werden, wie es z.B. in der WO 01/36364 beschrieben ist, wobei im ersten Zweizonenteil die Propenoxidation und im zweiten Zweizonenteil die erfindungsgemäße Acroleinoxidation durchgeführt wird.

Normalerweise befindet sich im zweiten Fall zwischen den Festbettkatalysatorschüttungen für die beiden Reaktionsstufen eine Inertschüttung. Allerdings kann auf eine solche Zwischeninertschüttung auch verzichtet werden.

Natürlich kann die Propenoxidationsstufe aber auch ein separater oder mit der nachfolgenden Acroleinoxidationsstufe in entsprechender Weise verschmolzener Einzonenrohrbündelreaktor sein.

Die Länge der Reaktionsrohre entspricht im Verschmelzungsfall vielfach der Summe der Längen der nicht verschmolzenen Rohrbündelreaktoren.

Der Propenanteil im Reaktionsgasausgangsgemisch für die dem erfindungsgemäßen Verfahren vorausgehende Reaktionsstufe kann dabei z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das dem erfindungsgemäßen Verfahren vorausgehende Verfahren bei einem Propen : Sauerstoff: indifferente Gase (einschließlich Wasserdampf) Volumenverhältnis im Reaktionsgasausgangsgemisch von 1 : (1,0 bis 3,0) : (5 bis 25), vorzugsweise 1 : (1,4 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zur ≥ 80 Vol.-%, oder zu ≥ 90 Vol.-%, oder zu ≥ 95 Vol.-% aus molekularem Stickstoff bestehen (generell sollen in dieser Schrift inerte Verdünnungsgase solche sein ,die sich beim einmaligen Durchgang durch die jeweilige Reaktionsstufe zu weniger als 5 %, bevorzugt zu weniger als 2 % umsetzen; das sind neben molarem Stickstoff z.B. Gase wie Propan, Ethan, Methan, Pentan, Butan, CO₂, CO, Wasserdampf und/oder Edelgase). Natürlich kann das inerte Verdünnungsgas beim dem erfindungsgemäßen Verfahren vorausgehenden Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung der Acrylsäure aus dem Produktgasgemisch des erfindungsgemäßen Verfahrens verbleibt.

Als Katalysatoren für eine solche gasphasenkatalytische Propenoxidation kommen insbesondere jene der EP - A 15565, der EP - A 575897, der DE - A 19746210 und der DE - A 19855913 in Betracht.

Der Acroleinanteil im Reaktionsgasausgangsgemisch für das erfindungsgemäße Verfahren kann z.B. bei Werten von 3 bis 15 Vol.-%, häufig bei 4 bis 10 Vol.-% bzw. 5 bis 8 Vol.-% liegen (jeweils bezogen auf das Gesamtvolumen).

Häufig wird man das erfindungsgemäße Verfahren mit einem im Reaktionsgasausgangsgemisch vorliegenden Acrolein : Sauerstoff: Wasserdampf: Inertgas - Volumenverhältnis (NI) von 1 : (1 bis 3): (0 bis 20): (3 bis 30), vorzugsweise von 1 : (1 bis 3) : (0,5 bis 10): (7 bis 10) ausführen.

Selbstredend kann man das erfindungsgemäße Verfahren aber auch mit einem im Reaktionsgasausgangsgemisch vorliegenden Acrolein : Sauerstoff: Wasserdampf: Sonstige- Volumenverhältnis (NI) von 1 : (0,9 bis 1,3): (2,5 bis 3,5): (10 bis 12) ausführen.

An dieser Stelle sei noch festgehalten, dass als Aktivmassen für die Festbettkatalysatorschüttung des erfindungsgemäßen Verfahrens auch die Multimetalloxidmassen der DE-A 10261186 günstig sind.

Generell lässt sich die volumenspezifische Aktivität zwischen zwei Festbettkatalysatorschüttungszonen experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Acrolein enthaltende Reaktionsgasausgangsgemisch geführt wird. Die höhere umgesetzte Menge an Acrolein weist die höhere volumenspezifische Aktivität aus.

Erfindungsgemäß günstige Ausgestaltungen eines Zweizonenrohrbündelreaktors für eine der erfindungsgemäßen Reaktionsstufe vorausgehende Propenpartialoxidationsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

### Kontaktrohre:

| | |
|---|---|
| Material der Kontaktrohre: | ferritischer Stahl; |
| Abmessungen der Kontaktrohre: | z.B. 3500 mm Länge; |
| | z.B. 30 mm Außendurchmesser; |
| | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

### Reaktor (Material wie die Kontaktrohre):

Zylinderförmiger Behälter eines Innendurchmessers von 6000 - 8000 mm;
Reaktorhauben plattiert mit Edelstahl des Typs 1,4541; Plattierungsdicke: einige mm;
ringförmig angeordnetes Rohrbündel, z.B. mit einem freien zentralen Raum;
Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);
normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z.B. 36 mm, oder 38 mm, oder 40 mm, oder 42 mm, oder 44 mm;
die Kontaktrohre sind mit ihren Enden in Kontaktrohrböden (oberer Boden und unterer Boden z.B. mit einer Dicke von 100 - 200 mm) abdichtend befestigt und münden am oberen Ende in eine mit dem Behälter verbundene Haube, die einen Zulass für das Reaktionsgasausgangsgemisch aufweist; ein z.B. auf der halben Kontaktrohrlänge befindliches Trennblech einer Dicke von 20 - 100 mm, teilt den Reaktorraum symmetrisch in zwei Reaktionszonen (Temperaturzonen) A' (obere Zone) und B' (untere Zone); jede Reaktionszone wird durch eine Umlenkscheibe in 2 äquidistante Längsabschnitte geteilt;
die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;
jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;
aus beiden Salzschmelzekreisen wird z.B. ein Teilstrom entnommen und z.B. in einem gemeinsamen oder zwei getrennten indirekten Wärmetauschern abgekühlt (Dampferzeugung);
im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;
durch im Reaktormantel befindliche Fenster gelangt die Salzschmelze zum Rohrbündel; die Einströmung erfolgt z.B. in radialer Richtung zum Rohrbündel;
die Salzschmelze fließt in jeder Zone der Vorgabe des Umlenkblechs folgend z.B. in der Abfolge
- von außen nach innen,
- von innen nach außen,
um die Kontaktrohre;
durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;
über jede Reaktionszone wird die Salzschmelze von unten nach oben geführt.

Das Reaktionsgasgemisch verlässt den Reaktor der der erfindungsgemäßen Reaktionsstufe voraus geschalteten Reaktionsstufe mit einer Temperatur wenige Grad höher als die Salzbadeintrittstemperatur dieses Reaktors. Das Reaktionsgasgemisch wird für die Weiterverarbeitung zweckmäßigerweise in einem separaten Nachkühler, der diesem Reaktor nachgeschaltet ist, auf 220°C bis 280°C, bevorzugt 240°C bis 260°C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

### Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 m³/h betragen.

### Stromführung:

das Reaktionsgasausgangsgemisch für die Propenoxidation strömt zweckmäßig von oben nach unten durch den Reaktor, während die unterschiedlich temperierten Salzschmelzen der einzelnen Zonen zweckmäßig von unten nach oben gefördert werden;
Kontaktrohr- und Thermorohrbeschickung (von oben nach unten) z.B.:
- Abschnitt 1:: 50 cm Länge
Steatitringe der Geometrie 7 mm x 7 mm x 4 mm (Außendurchmesser x Länge x Innendurchmesser) als Vorschüttung.
- Abschnitt 2:: 140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.
- Abschnitt 3:: 160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2 WO₃]_{0,5} [Mo₁₂Co_{5,5}Fe_{2,94}Si_{1,59}K_{0,08}Oₓ]₁).

Erfindungsgemäß günstige Ausgestaltungen eines Zweizonenrohrbündelreaktors für die erfindungsgemäße Acroleinreaktionsstufe können wie folgt beschaffen sein:
Alles wie beim Zweizonenrohrbündelreaktor für die Propenreaktionsstufe. Die Dicke des oberen und unteren Kontaktrohrbodens beträgt jedoch häufig 100 - 200 mm, z.B. 110 mm, oder 130 mm, oder 150 mm, oder 170 mm, oder 190 mm.

Der Nachkühler entfällt; statt dessen münden die Kontaktrohre mit ihren unteren Öffnungen in eine am unteren Ende mit dem Behälter verbundene Haube mit Auslass für das Produktgasgemisch; die obere Reaktionszone ist die Zone A und die untere Reaktionszone ist die Reaktionszone B. Zwischen Auslass "Nachkühler" und Einlass "Reaktor für die erfindungsgemäße Reaktionsstufe" besteht zweckmäßig eine Zufuhrmöglichkeit für komprimierte Luft.

Die Kontaktrohr- und Thermorohrbeschickung (von oben nach unten) kann z.B. wie folgt sein:
- Abschnitt 1:: 20 cm Länge
Steatitringe der Geometrie 7 mm x 7 mm x 4 mm (Außendurchmesser x Länge x Innendurchmesser) als Vorschüttung.
- Abschnitt 2:: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.
- Abschnitt 3:: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.
- Abschnitt 4:: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

Alternativ kann die Propenstufen-Kontaktrohr- und Thermorohrbeschickung (von oben nach unten) auch so aussehen:
- Abschnitt 1:: 50 cm Länge
Steatitringe der Geometrie 7 mm x 7 mm x 4 mm (Außendurchmesser x Länge x Innendurchmesser) als Vorschüttung.
- Abschnitt 2:: 300 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} • [Mo₁₂Co_{5,6}Fe_{2,94}Si_{1,59}K_{0,08}Oₓ]₁).

Die Acroleinstufen-Kontaktrohr- und Thermorohrbeschickung kann (von oben nach unten) auch so aussehen:
- Abschnitt 1:: 20 cm Länge
Steatitringe der Geometrie 7 mm x 7 mm x 4 mm (Außendurchmesser x Länge x Innendurchmesser) als Vorschüttung.
- Abschnitt 2:: 140 cm Länge
Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.
- Abschnitt 3:: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

In allen genannten Propenstufenbeschickungen kann der Vollkatalysator aus Beispiel 1 der DE-A 10046957 auch ersetzt werden durch:
a) einen Katalysator gemäß Beispiel 1 c aus der EP-A 15565 oder einen gemäß diesem Beispiel herzustellenden Katalysator, der jedoch die Aktivmasse Mo₁₂Ni_{6,5}Zn₂Fe₂Bi₁P_{0,0065}K_{0,06}Oₓ • 10 SiO₂ aufweist;
b) Beispiel Nr. 3 aus der DE-A 19855913 als Hohlzylindervollkatalysator der Geometrie 5 mm x 3 mm x 2 mm bzw. 5 mm x 2 mm x 2 mm;
c) Multimetalloxid II - Vollkatalysator gemäß Beispie 1 der DE-A 19746210;
d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht;
e) einen Schalenkatalysator gemäß den Ausführungsbeispielen der DE-A 19815281 jedoch bei gleicher Schalendicke auf Trägerringe der Geometrie 7 mm x 3 mm x 4 mm, oder 8 mm x 6 mm x 4 mm (stets Außendurchmesser x Länge x Innendurchmesser) aufgebracht.

In vorgenannter Auflistung wird als Trägermaterial bevorzugt Steatit der Fa. CeramTec eingesetzt (Typ: C220).

In allen vorgenannten erfindungsgemäßen Acroleinstufenbeschickungen kann der Schalenkatalysator gemäß Herstellungsbeispiel 5 der DE-A 10046928 ersetzt werden durch:
a) Schalenkatalysator S1 oder S7 aus der DE-A 4442346 mit einem Aktivmassenanteil von 27 Gew.-% und einer Schalendicke von 230 µm;
b) einem Schalenkatalysator gemäß den Beispielen 1 bis 5 aus der DE-A 19815281, jedoch auf Trägerring der Geometrie 7 mm x 3 mm x 4 mm mit einem Aktivmassenanteil von 20 Gew.-% aufgebracht;
c) Schalenkatalysator mit zweiphasiger Aktivmasse der Stöchiometrie (Mo_{10,4}V₃W_{1,2}Qₓ) (CuMo_{0,5}W_{0,6}O₄)_{1,6}, hergestellt gemäß DE-A 19736105 und mit einem Aktivmassenanteil von 20 Gew.-% auf den vorgenannten 7 mm x 3 mm x 4 mm Träger aufgebracht.

Im Übrigen werden die Festbettkatalysatorschüttung für die Propenoxidationsstufe und die Festbettkatalysatorschüttung für die erfindungsgemäße Acroleinoxidationsstufe zweckmäßig so gewählt (z.B. durch Verdünnung mit z.B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperaturunterschied ≤ 70°C, häufig liegt er bei 20 bis 70°C, vorzugsweise ist dieser Temperaturunterschied gering. Außerdem werden diese Festbettkatalysatorschüttungen aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 1106598 ≤ 9°C, oder ≤ 7°C, oder ≤ 5°C, oder ≤ 3°C beträgt.

Nachkühler und Reaktor für die Acroleinreaktionsstufe sind durch ein Verbindungsrohr verbunden, dessen Länge weniger als 25 m beträgt.

Die beschriebene Reaktoranordnung und alle anderen in dieser Schrift beschriebenen Reaktoranordnungen und Beschickungen mit Festbettkatalysator können auch bei hohen Propen- bzw. Acroleinlasten wie in den Schriften WO 01/36364, DE-A 19927624, DE-A 19948248, DE-A 19948523, DE-A 19948241, DE-A 19910506, DE-A 10302715 und EP-A 1106598 beschrieben betrieben werden.

Bevorzugt können die Reaktionszonen A', B' bzw. A, B dabei die in dieser Schrift empfohlenen Temperaturen aufweisen, jedoch so, dass die jeweils zweite Reaktionszone, gemäß der Lehre der vorgenannten Schriften, eine höhere Temperatur als die jeweils erste Reaktionszone aufweist. Die Heißpunkttemperatur in der jeweils zweiten Reaktionszone liegt vorzugsweise stets unterhalb derjenigen der jeweils ersten Reaktionszone.

Bei den erfindungsgemäßen Acroleinlasten resultiert jedoch mit der erfindungsgemäßen Verfahrensweise eine, relativ zu der für hohe Acroleinlasten empfohlenen Verfahrensweise, erhöhte Selektivität der Acrylsäurebildung.

In den nachfolgenden Beispielen und Vergleichsbeispielen sowie in der vorstehenden Reaktoranordnung können in der Acroleinreaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden. Die Aussage bezüglich des Betriebs bei hohen Acroleinlasten gemäß den vorgenannten Schriften behält dabei ihre Gültigkeit.

### Beispiele und Vergleichsbeispiele

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1:: 20 cm Länge
Steatitringe der Geometrie 7 mm x 7 mm x 4 mm (Außendurchmesser x Länge x Innendurchmesser) als Vorschüttung.
- Abschnitt 2:: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschitt 4.
- Abschnitt 3:: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.
- Abschnitt 4:: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

### Gasphasenoxidation:

Das vorstehend beschriebene Reaktionsrohr wird mit einem Reaktionsgasausgangsgemisch der nachfolgenden Zusammensetzung kontinuierlich beschickt, wobei die Belastung und die Thermostatisierung des Reaktionsrohres variiert werden:
5,5 Vol.-% Acrolein,
0,3 Vol.-% Propen,
6,0 Vol.-% molekularer Sauerstoff,
0,4 Vol.-% CO,
0,8 Vol.-% CO₂,
9,0 Vol.-% Wasser und,
78,0 Vol.-% Stickstoff.

Das Reaktionsgasgemisch durchströmt das Reaktionsrohr von oben nach unten.

Der Druck am Eingang des Reaktionsrohres variiert in Abhängigkeit von der Acroleinbelastung zwischen 1,6 und 2,1 bar.

Dem Produktgasgemisch wird am Reaktionsrohrausgang eine kleine Probe für die gaschromatographische Analyse entnommen. Am Ende der Reaktionszone A befindet sich ebenfalls eine Analysenstelle.

Die Ergebnisse in Abhängigkeit von Belastungen und Salzbadtemperaturen zeigt die nachfolgende Tabelle (der Buchstabe B in Klammern bedeutet Beispiel und der Buchstabe V in Klammern bedeutet Vergleichsbeispiel).
- T_{A},T_{B},: stehen für die Temperaturen der umgepumpten Salzbäder in den Reaktionszonen A und B.
- U_{AA}: ist der Acroleinumsatz am Ende der Reaktionszone A in mol-%.
- U_{AB}: ist der Acroleinumsatz am Ende der Reaktionszone B in mol-%.
- S^{AA}: ist die Selektivität der Acrylsäurebildung im Produktgasgemisch bezogen auf umgesetztes Acrolein in mol-%.
- T^{maxA}, T^{maxB}: stehen für die höchste Temperatur des Realctionsgasgemisches innerhalb der Reaktionszonen A und B in °C.

**Tabelle**

| Acroleinbeiastung (Nl/l•h) | T_{A} | T_{B} | T^{maxA} | T^{maxB} | U_{AA} | U_{AB} | S^{AA} |
|---|---|---|---|---|---|---|---|
| 106 (B) | 260 | 260 | 302 | 276 | 80,7 | 99,3 | 95,4 |
| 108 (B) | 262 | 259 | 312 | 275 | 84,8 | 99,3 | 95,8 |
| 104 (V) | 257 | 262 | 285 | 291 | 64,0 | 99,3 | 94,9 |
| 152 (V) | 263 | 269 | 303 | 287 | 78,8 | 99,3 | 95,8 |
| 147 (V) | 257 | 278 | 278 | 310 | 61,3 | 99,3 | 95,0 |

## Patentansprüche

1. Ein Verfahren der heterogen katalysierten partiellen Gasphasenoxidation von Acrolein zu Acrylsäure, bei dem man ein Acrolein, molekularen Sauerstoff und wenigstens ein Inertgas, das zu wenigstens 20% seines Volumens aus molekularem Stickstoff besteht, enthaltendes Reaktionsgasausgangsgemisch, das den molekularen Sauerstoff und das Acrolein in einem molaren Verhältnis O₂:C₃H₄O ≥ 0,5 enthält, in einer Reaktionsstufe so über eine Festbettkatalysatorschüttung, die in zwei räumlich aufeinander folgenden Reaktionszonen A.B angeordnet ist, wobei die Temperatur der Reaktionszone A eine Temperatur im Bereich von 230 bis 320°C und die Temperatur der Reaktionszone B ebenfalls eine Temperatur im Bereich von 230 bis 320°C ist, und deren Aktivmasse wenigstens ein die Elemente Mo und V enthaltendes Multimetalloxid ist, führt, dass sich die Reaktionszone A bis zu einem Umsatz des Acroleins von 45 bis 85 mol-% erstreckt und bei einmaligem Durchgang des Reaktionsgasausgangsgemischs durch die gesamte Festbettkatalysatorschüttung der Acroleinumsatz ≥ 90 mol-% und die Selektivität der Acrylsäurebildung, bezogen auf umgesetztes Acrolein, ≥ 90 mol-% beträgt, wobei die zeitliche Abfolge, in der das Reaktionsgasausgangsgemisch die Reaktionszonen durchströmt, der alphabetischen Abfolge der Reaktionszonen entspricht, das **dadurch gekennzeichnet ist, dass**
a) die Belastung der Festbettkatalysatorschüttung mit dem im Reaktionsgasausgangsgemisch enthaltenen Acrolein ≤ 125 Nl Acrolein/l Festbettkatalysatorschüttung h und ≥ 70 Nl Acrolein/l Festbettkatalysatorschüttung h beträgt,
b) die volumenspezifische Aktivität der Festbettkatalysatorschüttung in Strömungsrichtung des Reaktionsgasgemisches über die Festbettkatalysatorschüttung entweder konstant ist oder wenigstens einmal zunimmt, und
c) die Differenz T^{maxA} - T^{maxB}, gebildet aus der höchsten Temperatur T^{maxA}, die das Reaktionsgasgemisch innerhalb der Reaktionszone A aufweist, und der • höchsten Temperatur T^{maxB}, die das Reaktionsgasgemisch innerhalb der Reaktionszone B aufweist, ≥ 0°C beträgt.

2. Verfahren nach Anspruch 1, **dadurch gekennzeichnet, dass** die Differenz T^{maxA} - T^{maxB} ≥ 0°C und ≤ 75°C beträgt.

## Claims

1. A process for partially oxidizing acrolein to acrylic acid in the gas phase under heterogeneous catalysis by conducting a starting reaction gas mixture which comprises acrolein, molecular oxygen and at least one inert gas containing at least 20% by volume of molecular nitrogen and contains the molecular oxygen and the acrolein in a molar O₂:C₃H₄O ratio of ≥ 0.5 in one reaction stage over a fixed catalyst bed which is arranged in two spatially successive reaction zones A,B, the temperature of reaction zone A being a temperature in the range from 230 to 320°C and the temperature of reaction zone B likewise being a temperature in the range from 230 to 320°C, whose active composition is at least one multimetal oxide comprising the elements Mo and V, in such a way that reaction zone A extends to an acrolein conversion of from 45 to 85 mol% and, on single pass of the starting reaction gas mixture through the overall fixed catalyst bed, the acrolein conversion is ≥ 90 mol% and the selectivity of acrylic acid formation, based on acrolein converted, is ≥ 90 mol%, the chronological sequence in which the starting reaction gas mixture flows through the reaction zones corresponding to the alphabetic sequence of the reaction zones, wherein
a) the hourly space velocity of the acrolein contained in the starting reaction gas mixture on the fixed catalyst bed is ≤ 125 1 (STP) of acrolein/l of fixed catalyst bed·h and ≥ 70 1 (STP) of acrolein/l of fixed catalyst bed·h,
b) the volume-specific activity of the fixed catalyst bed is either constant or increases at least once in the flow direction of the reaction gas mixture over the fixed catalyst bed, and
c) the difference T^{maxA} - T^{maxB}, formed from the highest temperature T^{maxA} which the reaction gas mixture has within the reaction zone A and the highest temperature T^{maxB} which the reaction gas mixture has within reaction zone B, is ≥ 0°C.

2. The process according to claim 1, wherein the difference T^{maxA} - T^{maxB} is ≥ 0°C and ≤ 75°C.

## Revendications

1. Procédé pour l'oxydation partielle, catalysée par voie hétérogène, en phase gazeuse, d'acroléine en acide acrylique, dans lequel on fait passer un mélange réactionnel gazeux initial contenant de l'acroléine, de l'oxygène moléculaire et au moins un gaz inerte, qui est constitué à raison d'au moins 20% de son volume d'oxygène moléculaire, qui contient l'oxygène moléculaire et l'acroléine dans un rapport molaire O₂:C₃H₄O ≥ 0,5, en une étape de réaction sur un catalyseur en lit fixe en vrac, qui est disposé dans deux zones de réaction A, B consécutives dans l'espace, la température de la zone de réaction A étant une température dans la plage de 230 à 320°C et la température de la zone de réaction B étant également une température dans la plage de 230 à 320°C, et dont la masse active est au moins un oxyde multimétallique contenant les éléments Mo et V, de manière telle que la zone de réaction A s'étend jusqu'à une conversion de l'acroléine de 45 à 85% en mole et que, lors du passage unique du mélange réactionnel gazeux initial au travers de l'ensemble du catalyseur en lit fixe en vrac, la conversion de l'acroléine est ≥ 90% en mole et la sélectivité de la formation de l'acide acrylique, par rapport à l'acroléine transformée, est ≥ 90%, l'ordre temporel dans lequel le mélange réactionnel gazeux initial s'écoule au travers des zones de réaction correspondant à l'ordre alphabétique des zones de réaction, **caractérisé en ce que**
a) la charge du catalyseur en lit fixe en vrac par l'acroléine contenue dans le mélange réactionnel gazeux initial est ≤ 125 Nl d'acroléine/l de catalyseur en lit fixe en vrac*h et ≥ 70 Nl d'acroléine/l de catalyseur en lit fixe en vrac*h,
b) l'activité volumique spécifique du catalyseur en lit fixe en vrac dans le sens d'écoulement du mélange gazeux réactionnel sur le catalyseur en lit fixe en vrac soit est constante, soit augmente au moins une fois ; et
c) la différence T^{maxA}-T^{maxB}, formée à partir de la température la plus élevée T^{maxA} que le mélange gazeux réactionnel présente dans la zone de réaction A et de la température la plus élevée T^{maxB} que le mélange gazeux réactionnel présente dans la zone de réaction B, est ≥ 0°C.

2. Procédé selon la revendication 1, **caractérisé en ce que** la différence T^{maxA}-T^{maxB} est ≥ 0°C et ≤ 75°C.
